(19) Europäisches Patentamt
European Patent Office
Office européen des brevets

(11) Publication number: **0 480 152 B1**

(12) **EUROPEAN PATENT SPECIFICATION**

(45) Date of publication of patent specification: **11.01.95**

(51) Int. Cl.[6]: **C07H 15/26**, A61K 31/71, C07D 311/78, C07D 493/04, A61K 31/35, A61K 31/365, //(C07D493/04,311:00,307:00)

(21) Application number: **91114293.3**

(22) Date of filing: **26.08.91**

(54) **KS-505 derivatives.**

(30) Priority: **04.09.90 JP 234173/90**

(43) Date of publication of application:
**15.04.92 Bulletin 92/16**

(45) Publication of the grant of the patent:
**11.01.95 Bulletin 95/02**

(84) Designated Contracting States:
**DE FR GB IT**

(56) References cited:
**EP-A- 0 214 708**
**EP-A- 0 369 744**
**EP-A- 0 372 986**

**CHEMICAL ABSTRACTS, vol. 97, no. 19, 8th November 1982, page 484, abstract no. 160369h, Columbus, Ohio, US; J.W. VILLIGER et al.: "Phosphodiesterase inhibitors facilitate memory for passive avoidance conditioning", & BEHAV. NEURAL BIOL. 1981, 31(3), 354-9**

(73) Proprietor: **KYOWA HAKKO KOGYO KABUSHIKI KAISHA**
**6-1, Ohtemachi Itchome**
**Chiyoda-ku**
**Tokyo (JP)**

(72) Inventor: **Saito, Yutaka**
**3-9-13, Naka-machi**
**Machida-shi,**
**Tokyo (JP)**
Inventor: **Sano, Hiroshi**
**1-6-2, Nishikubo**
**Shimizu-shi,**
**Shizuoka-ken (JP)**
Inventor: **Nakanishi, Satoshi**
**3-9-9, Naka-machi**
**Machida-shi,**
**Tokyo (JP)**
Inventor: **Matsuda, Yuzuru**
**1-22-7, Nukui Minami-cho**
**Koganei-shi,**
**Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Inventor: **Kase, Hiroshi**
**3-35-18, Maehara-cho**
**Koganei-shi,**
**Tokyo (JP)**

(74) Representative: **Casalonga, Axel et al**
**BUREAU D.A. CASALONGA - JOSSE**
**Morassistrasse 8**
**D-80469 München (DE)**

## Description

Background of the Invention

The present invention relates to KS-505 derivatives and pharmaceutically acceptable salts thereof. The compounds exhibit an inhibitory activity on cyclic nucleotide phosphodiesterase and are expected to be useful as pharmaceuticals and intermediates thereof.

Cyclic nucleotide phosphodiesterase (hereinafter referred to as PDE) is an enzyme which decomposes cyclic adenosine-3',5'-monophosphate (hereinafter referred to as cAMP) and cyclic guanosine-3',5'-monophosphate (hereinafter referred to as cGMP) to form adenosine-5'-monophosphate and guanosine-5'-monophosphate, respectively.

cAMP and cGMP are important physiologically active substances which are considered to be concerned in the mechanism of contraction of smooth muscles of bronchus, blood vessels, etc., the mechanism of platelet aggregation, proliferation of cells, and the like.

It is known that a substance capable of inhibiting PDE increases the concentration of cAMP and cGMP in vivo and thereby exhibits a bronchodilative effect, a smooth muscle-relaxing effect, a cardiotonic effect, accelerating effect on hormone secretion, inhibitory effect on platelet aggregation, etc. Such a substance is thus useful as a pharmaceutical.

EP-0372986 discloses the compound KS-505 represented by formula (III):

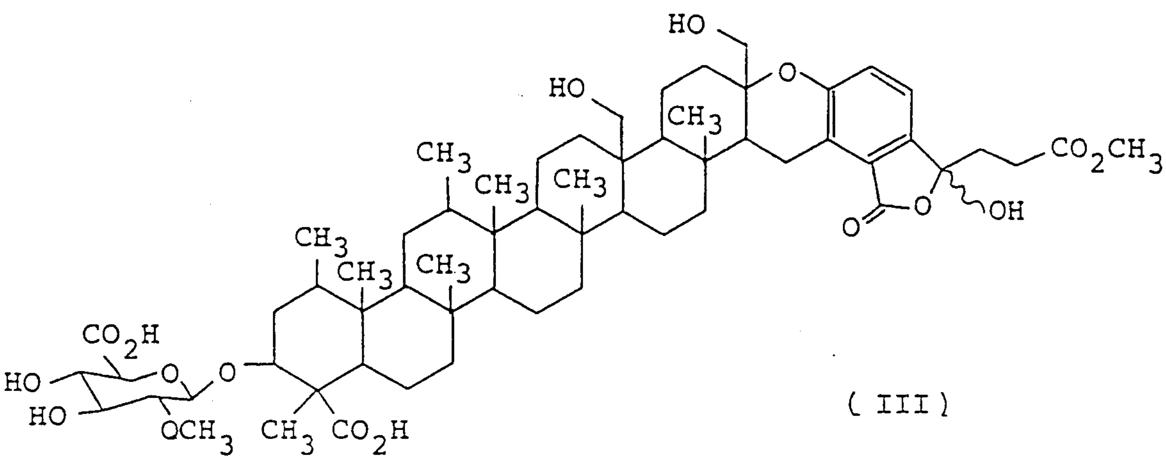

( III )

Summary of the Invention

The present invention provides KS-505 derivatives represented by formula (I):

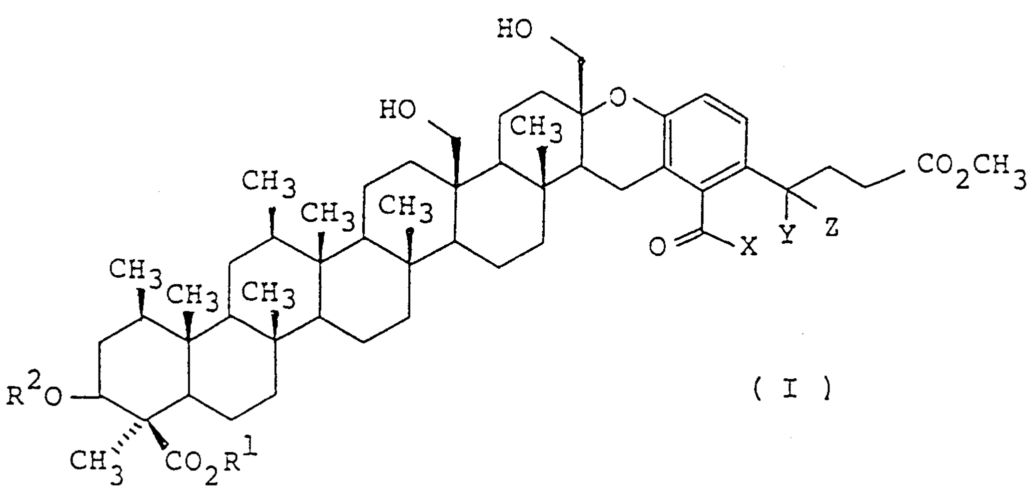

( I )

wherein $R^1$ represents hydrogen or methyl; $R^2$ represents hydrogen or a group of formula (II):

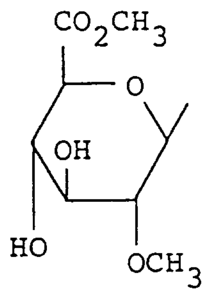

( II )

and X, Y and Z represent the following: when X is $OCH_3$, Y and Z are combined together to form =O, and when Z is $OCH_3$, X and Y are combined together to form -O-; provided that when $R^1$ is hydrogen, $R^2$ represents hydrogen
and pharmaceutically acceptable salts thereof.

Detailed Description of the Invention

Examples of the compounds represented by formula (I) [hereinafter referred to as Compounds (I)] are shown in Tables 1 and 2.

Table 1

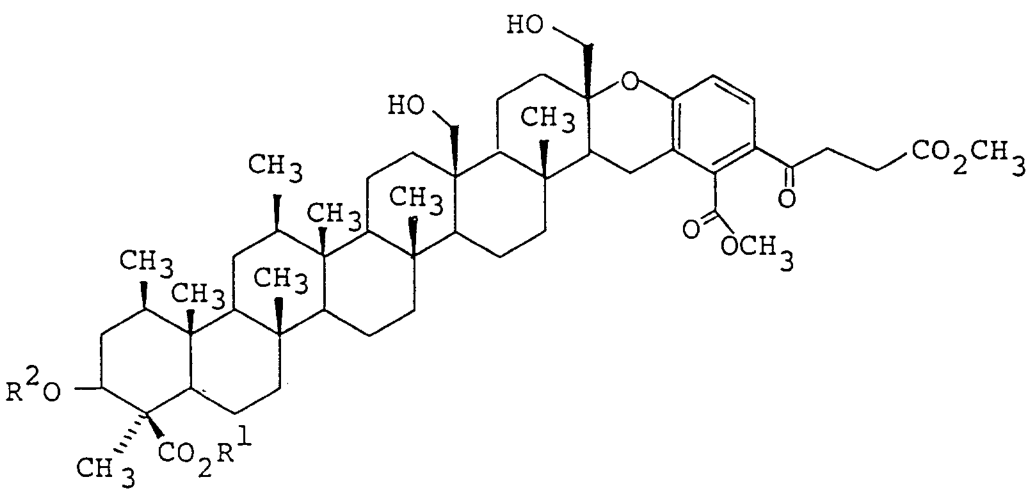

| Compound | $R^1$ | $R^2$ |
|---|---|---|
| 1 | $CH_3$ | formula (II) |
| 2 | $CH_3$ | H |
| 3 | H | H |

Table 2

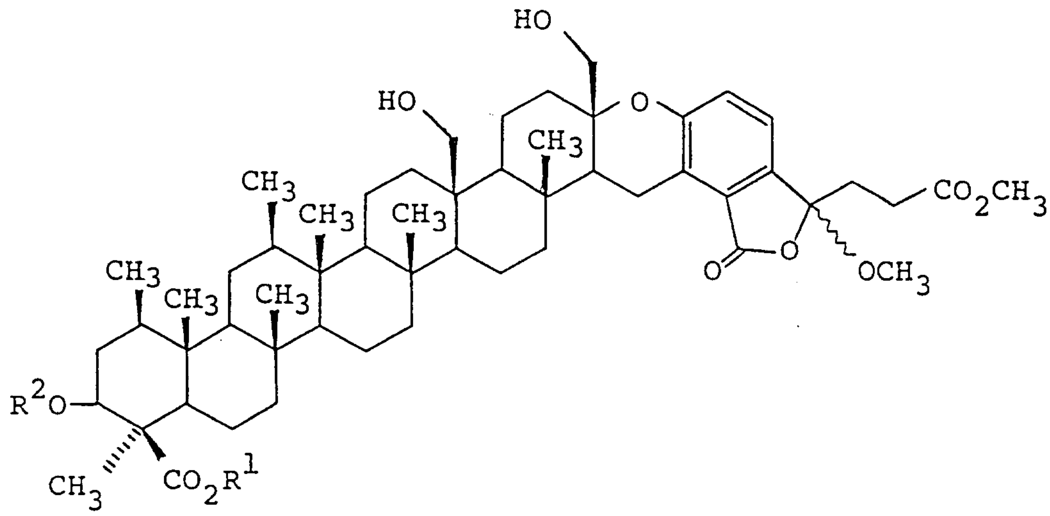

| Compound | $R^1$ | $R^2$ |
|---|---|---|
| 4 | $CH_3$ | formula (II) |
| 5 | $CH_3$ | H |
| 6 | H | H |

As the pharmaceutically acceptable salts of KS-505 derivatives, those formed with suitable organic or inorganic bases may be mentioned. Examples of suitable organic bases include primary amines such as methylamine, ethylamine and aniline; secondary amines such as dimethylamine, diethylamine, pyrrolidine, piperidine, morpholine and piperazine; and tertiary amines such as trimethylamine, triethylamine, N,N-dimethylaniline and pyridine. Examples of suitable inorganic bases are alkali metals such as sodium and potassium; and alkaline earth metals such as magnesium and calcium.

The physicochemical properties of KS-505 derivatives are shown in Tables 3 and 4 below.

The data were obtained by using the following equipments.

NMR : Bruker AM-400

MS : Hitachi M-80B (SIMS method, positive and negative modes)

IR : Shimadzu IR-27G, $CHCl_3$ solution method

mp : Yanagimoto's micro melting point apparatus

TLC : thin layer chromatography [Art 5744, Merck Inc., developing solvent; chloroform : methanol (20 : 1 v/v)]

Table 3

| Compound | SIMS | Melting Point TLC | IR ($cm^{-1}$) |
|---|---|---|---|
| 1 | $C_{64}H_{94}O_{17}$ (1134)<br>m/z 1135 ($M^+$+H) 1104 ($M^+$+H-$CH_3OH$) | 165-166 °C<br>Rf = 0.19 | 3500, 2950, 1730, 1675, 1585, 1440, 1385, 1350, 1320, 1280, 1165, 1045, 1010, 995, 980 |
| 2 | $C_{56}H_{82}O_{11}$ (930)<br>m/z 931 ($M^+$+H) 899 ($M^+$+H-$CH_3OH$) | 207-208 °C<br>Rf = 0.39 | 3550, 2950, 1730, 1585, 1440, 1390, 1350, 1320, 1280, 1160, 1140, 1095, 1050, 1010, 1000 |
| 3 | $C_{55}H_{80}O_{11}$ (916)<br>m/z 917 ($M^+$+H) | >360 °C<br>Rf = 0.29 | 3400, 2950, 1730, 1680, 1580, 1465, 1440, 1320, 1280, 1160, 1135, 1095, 1010, 995 |
| 4 | $C_{64}H_{94}O_{17}$ (1134)<br>m/z 1104 ($M^+$+H-$CH_3OH$) | 259-260 °C<br>Rf = 0.25 | 3550, 2950, 1730, 1585, 1440, 1390, 1350, 1320, 1280, 1160, 1140, 1095, 1050, 1010, 1000 |
| 5 | $C_{56}H_{82}O_{11}$ (930)<br>m/z 899 ($M^+$+H-$CH_3OH$) | 241-243 °C<br>Rf = 0.47 | 3450, 2950, 1760, 1740, 1485, 1450, 1385, 1310, 1270, 1240, 1190, 1155, 1130, 1050, 1010, 995** |
| 6 | $C_{55}H_{80}O_{11}$ (916)<br>m/z 885 ($M^+$+H-$CH_3OH$) m/z 915 ($M^-$-H)* | 134-136 °C<br>Rf = 0.32 | 3400, 2950, 1755, 1735, 1480, 1445, 1385, 1320, 1270, 1160, 1130, 1050, 1035, 1010, 950, 900 |

Notes) * measured by negative mode
** measured by KBr tablet method

Table 4

| Compound | $^{13}C$ NMR 100 MHz Chloroform-d Solution |
|---|---|
| 1 | 10.7, 14.2, 15.6, 16.6, 16.8, 17.6, 18.0, 19.0, 19.6, 20.6, 21.4, 22.1, 22.2, 24.2, 28.1, 32.9, 33.5, 35.1, 36.8, 37.2, 38.1, 38.4, 38.6, 41.6 x3, 41.7, 41.8, 42.1, 42.7, 42.9, 49.6, 50.9, 51.8, 51.9, 52.6, 52.7, 57.7, 60.3, 60.7 x2, 61.9, 62.0, 62.8, 63.2, 63.4, 71.5, 73.9, 75.3, 80.5, 82.5, 87.3, 105.8, 117.6, 120.8, 127.1, 128.7, 136.0, 156.7, 169.6, 170.0, 173.2, 174.3, 196.6 |
| 2 | 10.4, 14.3, 15.6, 16.6, 16.8, 17.7, 18.0, 19.0, 19.9, 20.7, 21.5, 22.0, 22.2, 23.8, 28.1, 32.9, 33.6, 35.1, 36.8, 37.2, 38.2, 38.6, 40.4, 41.6, 41.7, 41.8 x2, 41.9, 42.5, 42.8 x2, 49.1, 51.1, 51.8, 51.9, 52.6, 57.5, 60.2, 60.4, 61.9, 62.1, 62.9, 63.3, 63.4, 77.0, 80.6, 117.6, 120.9, 127.2, 128.7, 136.0, 156.7, 169.6, 173.2, 178.2, 196.6 |
| 3 | 10.7, 14.3, 15.6, 16.6, 16.9, 17.7, 18.1, 19.1, 19.8, 20.7, 21.4, 22.0, 22.2, 24.2, 28.2, 33.0, 33.6, 35.2, 36.8, 37.2, 38.2, 38.7, 40.4, 41.7 x2, 41.9, 42.0 x2, 42.4, 42.8, 42.9, 48.7, 51.9 x2, 52.7, 57.6, 60.3 x2, 62.0, 62.1, 62.9, 63.2, 63.3, 76.7, 80.6, 117.6, 120.9, 127.1, 128.7, 136.0, 156.8, 169.8, 173.3, 179.7, 196.7 |

Table 4 (cont'd)

| Compound | $^{13}C$ NMR 100 MHz Chloroform-d Solution |
|---|---|
| 4 | 10.7, 14.2, 15.6, 16.6 x2, 17.6, 18.0, 18.6, 19.6, 20.6, 21.5, 22.1, 22.2, 24.2, 28.4, 32.9, 34.1, 35.2, 36.6, 37.2, 38.1, 38.4, 38.6, 41.6 x2, 41.7 x2, 41.8, 42.1, 42.8, 42.9, 49.6, 50.9, 50.9*, 51.1*, 51.6, 51.7, 52.7, 57.8, 59.9, 60.7 x2, 62.0, 62.2, 62.9, 63.3, 63.4, 71.5, 73.7, 75.3, 80.4, 82.5, 87.3, 105.8, 108.6, 121.1, 123.2, 123.7, 126.1, 138.5*, 138.6*, 154.4*, 154.5*, 167.8, 170.0, 173.1, 174.2 |
| 5 | 10.4, 14.3, 15.6, 16.6, 16.9, 17.7, 18.1, 18.6*, 18.7*, 19.9, 20.7, 21.5, 22.0, 22.2, 23.8, 28.4, 32.9, 34.2*, 34.3*, 35.3, 36.8, 37.3, 38.2, 38.6, 40.4, 41.7 x2, 41.8, 41.9 x2, 42.5, 42.8 x2, 49.0, 50.9, 51.1, 51.7 x2, 57.6, 59.9*, 60.0*, 60.2, 62.0*, 62.1*, 62.2*, 62.3*, 62.9, 63.3, 63.4, 77.0, 80.4*, 80.5*, 108.7, 121.1*, 121.2*, 123.2, 123.7*, 123.8*, 126.1, 138.5*, 138.6*, 154.5, 167.8, 173.1, 178.2 |
| 6** | 10.7, 14.3, 15.7, 16.6, 16.9, 17.7, 18.1, 18.6*, 18.7*, 19.8, 20.7, 21.5, 22.0, 22.2, 24.1, 28.4, 33.0, 34.2, 35.3, 36.8, 37.3, 38.2, 38.7, 40.3, 41.7, 41.9, 42.0, 42.4, 42.8, 42.9, 48.8, 50.9, 51.1, 51.7, 51.8, 57.6, 59.9, 60.3, 62.1, 62.2*, 62.3*, 62.9, 63.3 x2, 69.3, 77.4, 80.4, 108.7, 121.2, 123.2, 123.8, 126.1, 138.5*, 138.6*, 154.5*, 154.6*, 167.8*, 167.9*, 173.2, 180.8 |

Notes) * peak corresponding to α- or β-isomer of methoxy group

** solvent mixture of chloroform-d-methanol-$d_4$

The PDE-inhibiting activity of representative compounds is shown by Experimental Example.

Experimental Example

A PDE preparation partially purified from bovine cerebral cortex according to the method of Kakiuchi, et al. [Biochem. J., 146, 109-120 (1975)] was used as the PDE in the experiment. Fifty µl of a solution of a test compound in dimethylsulfoxide was added to 500 µl of a reaction mixture comprising 80 mM imidazole-hydrochloride buffer (pH 6.9), 3 mM magnesium sulfate, 0.3 mM dithiothreitol, 100 mM sodium chloride, 50 µM calcium chloride, 1.2 mM cAMP, 4 U/ml calmodulin (1 U = amount which gives 50% stimulation of PDE in the presence of 26 mU/ml PDE) and 26 mU/ml PDE (1 U = amount which hydrolyzes 1 µmol of cAMP in one minute).

The reaction was allowed to proceed at 30 °C for 30 minutes and then stopped by heating at 100 °C for 5 minutes. Then, 6 µmol of manganese chloride and 0.2 U of 5'-nucleotidase (1 U = amount which produces 1 µmol of phosphoric acid in one minute) were added to the reaction mixture, and the reaction was allowed to proceed at 30 °C for 30 minutes.

The reaction was stopped by addition of 3 ml of 10% perchloric acid. The formed inorganic phosphate was quantitatively determined according to the method of Ames [Methods in Enzymology, 8, 115-116 (1966), Academic Press].

Percent inhibition (I %) of PDE activity calculated according to the following equation is shown in Table 5.

Percent Inhibition = (A-B)/A x 100 (%)

A: the amount of inorganic phosphate formed in the absence of a test compound

B: the amount of inorganic phosphate formed in the presence of a test compound

Table 5

| Test Compound | I % (9 µg/ml) |
|---|---|
| KS-505 | 70% |
| Compound 1 | 48% |
| Compound 3 | 70% |
| Compound 6 | 54% |

The process for producing KS-505 derivatives is explained below.

KS-505, which is the starting material, is a known compound represented by formula (III) as described above. KS-505 has a structure of γ-hydroxy-γ-lactone type and may take a chemically equivalent structure represented by formula (IV) shown below. The compounds of formulae (III) and (IV) usually exist as an equilibrium mixture in a solution but its mixing ratio varies depending upon, e.g., acidity of a solvent.

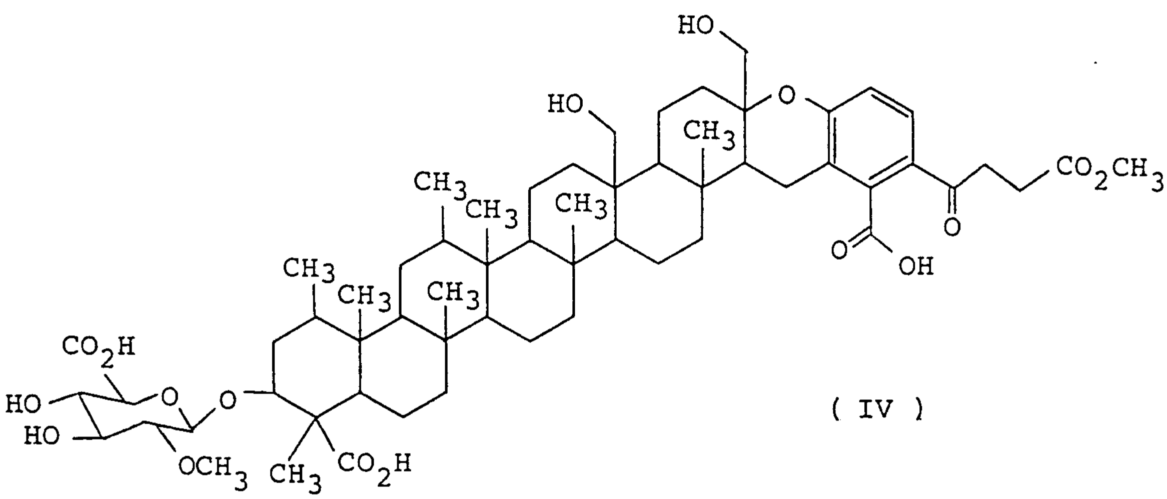

( IV )

The term KS-505 hereinafter refers to the compound of formula (III), the compound of formula (IV) or a mixture of the compounds of formulae (III) and (IV).

Compounds (I) can be prepared according to the following methods.

Method 1

Compounds Ia [Compounds (I) wherein $R^1$ is $CH_3$ and $R^2$ is a group of formula (II)] can be prepared by allowing KS-505 to react with diazomethane in the presence of an acid. As the acid, hydrogen chloride, acetic acid, p-toluenesulfonic acid, methanesulfonic acid, etc. may be used. The acid is used in an amount of 0.001 to 10 equivalents and diazomethane is used in an amount of 10 to 1000 equivalents, based on KS-505. The reaction is carried out at 0 to 30°C and is completed in 10 minutes to 15 hours.

Method 2

Compounds Ib [Compounds (I) wherein $R^1$ and $R^2$ are both H] can be prepared by treating KS-505 with an acid in methanol. As the acid, hydrogen chloride, sulfuric acid, phosphoric acid, etc. may be used. The concentration of the acid in the reaction solution is 0.1 to 2 N. The reaction is carried out at 30 to 70°C and is completed in 15 minutes to 15 hours.

Method 3

Compounds Ic [Compounds (I) wherein $R^1$ is $CH_3$ and $R^2$ is H] can be prepared in the same manner as in Method 1 except for the use of Compound Ib obtained in Method 2 instead of KS-505.

Method 4

Compounds Ic can also be prepared in the same manner as in Method 2 except for the use of Compound Ia obtained in Method 1 instead of KS-505.

In the reaction solution described above, KS-505 is usually an equilibrated mixture of the compound shown by formula (III) and the compound of formula (IV) which is chemically equivalent to the compound of formula (III). Therefore, Compounds Ia, Ib and Ic are usually obtained as mixtures of the compound having the structure of γ-methoxy-γ-lactone type wherein X and Y are combined together to form -O- and Z is $OCH_3$ and the compound having the structure of γ-ketocarboxylic acid methyl ester type wherein X is $OCH_3$ and Y and Z are combined together to form =O.

After completion of the reaction in each method described above, the reaction solution is concentrated, if necessary, after neutralization, and then extracted with a water-immiscible solvent such as ethyl acetate, chloroform or diethyl ether. The extract is washed with water, an aqueous solution of sodium hydrogencarbonate, a saturated aqueous solution of sodium chloride, or the like. After drying over anhydrous sodium sulfate, etc., the solvent is distilled off. Alternatively, the reaction solution is concentrated and the residue is purified by column chromatography using silica gel, thin layer chromatography, preparative high performance liquid chromatography, recrystallization, etc.

Certain specific embodiments of the present invention are illustrated in the following examples.

Example 1 Preparation of Compound 1

KS-505 (296 mg, 0.27 mmol) was dissolved in 90 ml of methanol, and 1 µl of acetic acid was added to the solution, followed by stirring. To the solution was added 25 ml of diazomethane-ether solution [bis(N-methyl-N-nitroso)terephthalamide (18 g)/ether (80 ml)]. The mixture was stirred at 25°C for 30 minutes and then the solvent was distilled off under reduced pressure. The resulting residue was purified by silica gel column chromatography [150 ml of silica gel, eluting solvent; chloroform : methanol = gradually changed from 20 : 1 to 10 : 1 (v/v)] to give 216 mg (70%) of Compound 1 as colorless powder.

Example 2 Preparation of Compounds 4 and 1

KS-505 (306 mg, 0.28 mmol) was dissolved in 45 ml of 0.3% hydrochloric acid-methanol. To the solution was added 30 ml of diazomethane-ether solution prepared in the same manner as in Example 1, and the mixture was stirred at 25°C for 2 hours. Purification was carried out in the same manner as in Example 1 to give 107 mg (33%) of Compound 4 as colorless powder and 49 mg (15%) of Compound 1 as colorless powder.

Example 3 Preparation of Compounds 5 and 2

Compound 1 (51.2 mg, 0.045 mmol) was dissolved in 30 ml of 0.1 N hydrochloric acid-methanol. The solution was heated to reflux for 14 hours, and then the solvent was distilled off under reduced pressure. The resulting residue was purified by preparative silica gel thin layer chromatography [Art 5744 (20 x 20 x 0.25 cm, manufactured by Merck Inc.), developing solvent; chloroform : methanol = 20 : 1 (v/v)] to give 21.6 mg (51%) of Compound 5 as colorless powder and 6.8 mg (16%) of Compound 2 as colorless powder.

Example 4 Preparation of Compounds 5 and 2

Compound 4 (4.2 mg, 3.7 µmol) was dissolved in 2 ml of 10% hydrochloric acid-methanol, and the solution was heated to reflux for 13 hours. Purification was carried out in the same manner as in Example 3 to give 2.6 mg (75%) of Compound 5 and 0.5 mg (15%) of Compound 2.

Example 5 Preparation of Compounds 5 and 2

A mixture (77 mg, 0.068 mmol) of Compounds 1 and 4 was dissolved in 10 ml of 10% hydrochloric acid-methanol. The solution was heated to reflux for 5 hours. Purification was carried out in the same manner as in Example 3 to give 34.6 mg (55%) of Compound 5 and 13.9 mg (22%) of Compound 2.

Example 6 Preparation of Compounds 6 and 3

KS-505 (200 mg, 0.18 mmol) was dissolved in 20 ml of 10% hydrochloric acid-methanol. The solution was heated to reflux for 7 hours, and then the solvent was distilled off under reduced pressure. The resulting residue was purified by silica gel column chromatography [100 ml of silica gel, eluting solvent; chloroform : methanol = gradually changed from 20 : 1 to 10 : 1 (v/v)] to give 72.9 mg (44%) of Compound 6 as colorless powder and 23.8 mg (14%) of Compound 3 as colorless powder.

Example 7 Preparation of Compound 5

Compound 6 (3.2 mg, 3.5 µmol) was dissolved in 1 ml of methanol. To the solution was added 1 ml of diazomethane-ether solution prepared in the same manner as in Example 1, and the mixture was stirred at 25°C for 40 minutes. Purification was carried out in the same manner as in Example 3 to give 2.7 mg (83%) of Compound 5.

**Claims**

**1.** A KS-505 derivative represented by formula (I):

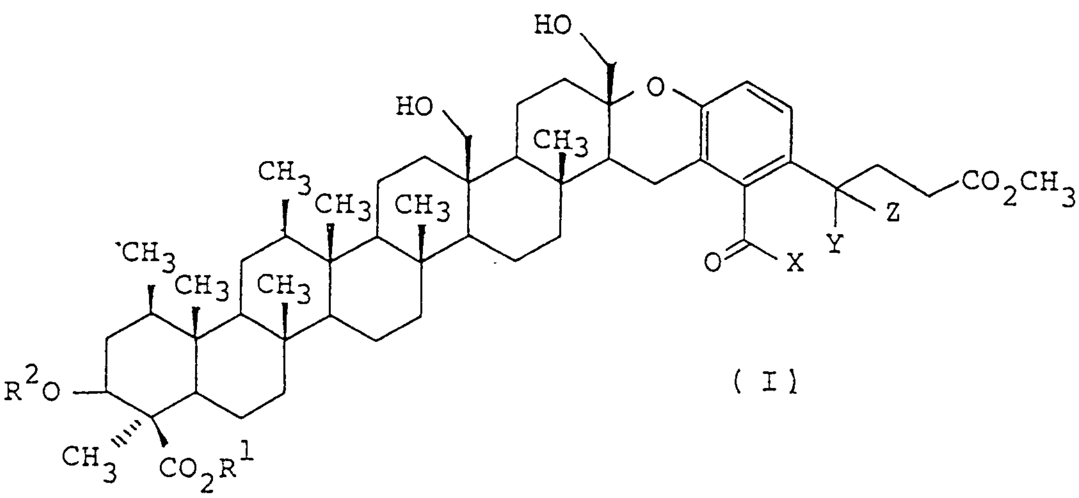

( I )

wherein $R^1$ represents hydrogen or methyl; $R^2$ represents hydrogen or a group of formula (II):

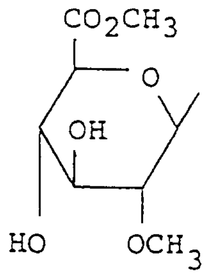

( II )

and X, Y and Z represent the following: when X is $OCH_3$, Y and Z are combined together to form =O, and when Z is $OCH_3$, X and Y are combined together to form -O-; provided that when $R^1$ is hydrogen, $R^2$ represents hydrogen, or a pharmaceutically acceptable salt thereof.

2. The compound according to Claim 1, wherein X is $OCH_3$ and Y and Z are combined together to form =O.

3. The compound according to Claim 1, wherein Z is $OCH_3$ and X and Y are combined together to form -O-.

4. The compound according to Claim 1, wherein the salt is formed with one member selected from the group consisting of methylamine, ethylamine, aniline, dimethylamine, diethylamine, pyrrolidine, piperidine, morpholine, piperazine, trimethylamine, triethylamine, N,N-dimethylaniline, pyridine, sodium, potassium, magnesium and calcium.

5. Compound according to any of claims 1-4 applicable as medicament.

6. Use of a compound according to any of claims 1-4 as a pharmaceutical or an intermediate therefor.

7. Use of a compound according to any of claims 1-4 for the manufacture of a medicament having PDE-inhibiting activity.

**Patentansprüche**

1. KS-505-Derivat der Formel (I)

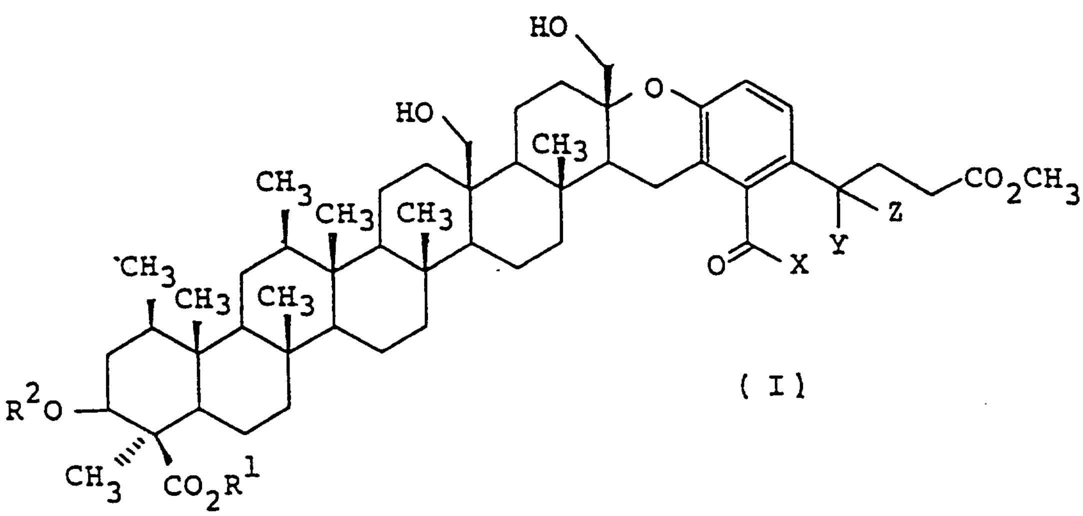

( I )

in der $R^1$ ein Wasserstoffatom oder eine Methylgruppe darstellt; $R^2$ ein Wasserstoffatom oder eine Verbindung der Formel (II) bedeutet:

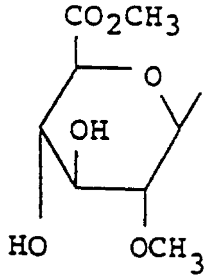

( II )

und X, Y und Z nachstehendes bedeuten: wenn X $OCH_3$ bedeutet, sind Y und Z zusammengenommen =O, und wenn Z $OCH_3$ bedeutet, sind X und Y zusammengenommen -O-; mit der Maßgabe, daß wenn $R^1$ ein Wasserstoffatom bedeutet, $R^2$ ein Wasserstoffatom wiedergibt, oder ein pharmazeutisch verträgliches Salz davon.

2. Verbindung nach Anspruch 1, wobei X $OCH_3$ bedeutet und Y und Z zusammengenommen =O darstellen.

3. Verbindung nach Anspruch 1, wobei Z $OCH_3$ bedeutet und X und Y zusammengenommen -O- bedeuten.

4. Verbindung nach Anspruch 1, wobei das Salz hergestellt wird mit Methylamin, Ethylamin, Anilin, Dimethylamin, Diethylamin, Pyrrolidin, Piperidin, Morpholin, Piperazin, Trimethylamin, Triethylamin, N,N-Dimethylanilin, Pyridin, Natrium, Kalium, Magnesium oder Calcium.

5. Verbindung nach einem der Ansprüche 1 bis 4, anwendbar als Arzneimittel.

6. Verwendung einer Verbindung nach einem der Ansprüche 1 bis 4 als Arzneimittel oder als Zwischenprodukt dafür.

7. Verwendung einer Verbindung nach einem der Ansprüche 1 bis 4 zur Herstellung eines Arzneimittels mit PDE-hemmender Wirkung.

## Revendications

1. Dérivé de KS-505 représenté par la formule (I) :

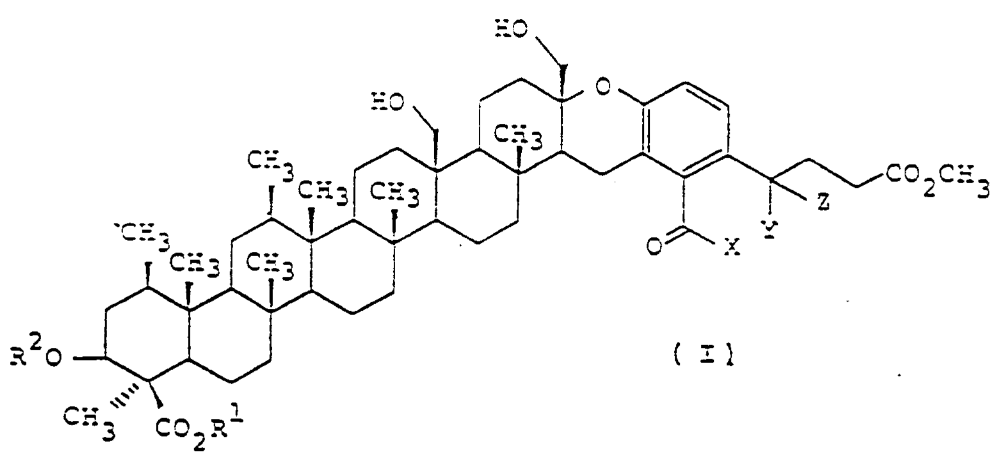

( I )

dans laquelle $R^1$ représente un atome d'hydrogène ou un groupe méthyle, $R^2$ représente un atome d'hydrogène ou un groupe de formule (II) :

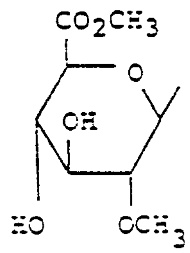

(II)

et X, Y et Z ont les significations suivantes : si X représente $-OCH_3$, Y et Z représentent ensemble =O ; et si Z représente $-OCH_3$, X et Y représentent ensemble -O- ; sous réserve que, lorsque $R^1$ représente un atome d'hydrogène, $R^2$ représente également un atome d'hydrogène ; ou sel pharmaceutiquement acceptable d'un tel dérivé.

2. Composé conforme à la revendication 1, dans lequel X représente $OCH_3$ et Y et Z forment ensemble =O.

3. Composé conforme à la revendication 1, dans lequel Z représente $OCH_3$ et X et Y forment ensemble -O-.

4. Composé conforme à la revendication 1, le sel étant formé avec un élément choisi dans l'ensemble constitué par la méthylamine, l'éthylamine, l'aniline, la diméthylamine, la diéthylamine, la pyrrolidine, la pipéridine, la morpholine, la pipérazine, la triméthylamine, la triéthylamine, la N,N-diméthylaniline, la pyridine, le sodium, le potassium, le magnésium et le calcium.

5. Composé conforme à l'une des revendications 1 à 4, utilisable comme médicament.

6. Emploi d'un composé conforme à l'une des revendications 1 à 4, en tant que produit pharmaceutique ou en tant qu'intermédiaire d'un tel produit.

7. Emploi d'un composé conforme à l'une des revendications 1 à 4 pour la fabrication d'un médicament présentant une activité d'inhibition de la PDE.